# EUROPÄISCHE PATENTSCHRIFT

(11) **EP 0 696 571 B1**
(45) Veröffentlichungstag und Bekanntmachung des Hinweises auf die Patenterteilung: **26.01.2000**
(21) Anmeldenummer: 95111996.5
(22) Anmeldetag: 31.07.1995
(51) Int. Cl.: C07C 205/06, C07C 201/08

(54) **Verfahren zur Herstellung von Dinitrotoluol**
Process for the preparation of dinitrotoluene
Procédé pour la préparation de dinitrotoluène

(30) Priorität: 11.08.1994 DE 4428462
(43) Veröffentlichungstag der Anmeldung: 14.02.1996
(73) Patentinhaber: BAYER AG, 51368 Leverkusen (DE)
(72) Erfinder: Klingler, Uwe, D-41539 Dormagen (DE); Schieb, Thomas, Dr., D-51503 Rösrath (DE); Wiechers, Gerhard, Dr., D-51381 Leverkusen (DE); Zimmermann, Jürgen, Dr., Walnut Creek, CA 94598 (US)

(56) Entgegenhaltungen:
- EP-A- 0 155 586
- EP-A- 0 597 361
- FR-A- 1 333 444
- US-A- 5 057 632

## Beschreibung

Die vorliegende Erfindung betrifft ein Verfahren zur zweistufigen Herstellung von Dinitrotoluol aus Toluol und Salpetersäure in Gegenwart von Schwefelsäure.

Dinitrotoluol (DNT) ist ein Vorprodukt zur Herstellung von Toluylendiisocyanat (TDI). Technisch wird DNT durch Umsetzung von Toluol mit Nitriersäure, einem Gemisch aus Salpeter- und Schwefelsäure gewonnen (DE-B 1 468 362; T. Urbanski, Chemistry and Technology of Explosives, Pergamon Press 1964; Ullmanns Encyklopädie der technischen Chemie, 4. Auflage, Bd. 17, S. 392, Verlag Chemie, Weinheim 1979). Dabei wird durch Umsetzung von Toluol mit einer verdünnten Nitriersäure zunächst Mononitrotoluol (MNT) hergestellt. Nach Abtrennung der gebrauchten Schwefelsäure (im folgenden als "Absäure" bezeichnet) wird dieses MNT in einer zweiten Stufe mit einer stärker konzentrierten Nitriersäure zu DNT umgesetzt. Beide Reaktionsstufen werden isotherm, d.h. unter Kühlung durchgeführt. Da die Nitrierung eine sehr stark exotherme Reaktion ist, ist der erforderliche Kühlaufwand hoch.

Beim zweistufigen Prozeß fallen zwei Absäuren an, die nach Aufstockung mit Salpetersäure wieder zurückgeführt werden. Die Rückführung ist bei der Absäure der 2. Stufe direkt möglich. Diese Absäure ist noch so konzentriert, daß sie ohne vorherige Aufkonzentrierung in der ersten Stufe wieder eingesetzt werden kann. Die Absäure aus der ersten Stufe bedarf dagegen einer Aufkonzentrierung zur Entfernung mindestens des Reaktionswassers, um wieder eingesetzt werden zu können. Die hierzu gebräuchlichsten Verfahren sind das Pauling-Verfahren (Bodenbrenner, von Plessen, Vollmüller, Dechema-Monogr. 86 (1980), 197) und die Eindampfung im Vakuum (Winnacker, Küchler, Chem. Technol., Bd. 2, Anorg. Technol. I, 4. Aufl., 1982, S. 70 bis 72). All diesen Verfahren ist gemeinsam, daß sie mit hohem Energieeinsatz arbeiten und demzufolge aufwendig und kostspielig sind.

Ein weiterer Nachteil des Verfahrens ist der vorzugsweise Einsatz von teurer, hochkonzentrierter Salpetersäure zur Wiederherstellung der Nitriersäure aus den Absäuren. Verdünnte Salpetersäurequalitäten wie Azeotrop- oder Schwachsäure, die deutlich preiswerter sind, können prinzipiell zwar verwendet werden, jedoch ist der zusätzliche Energieaufwand erheblich.

Die hier genannten Nachteile sind nicht spezifisch für die Nitrierung von Toluol, sondern treffen auch auf die Nitrierung anderer Aromaten zu.

Aus den oben genannten Gründen sind schon seit Jahren Bestrebungen im Gange, die Nitrierverfahren in den aufgeführten Punkten zu verbessern.

Durch adiabatische Reaktionsführung bei der Mono-Nitrierung von Benzol gelang eine Verbesserung in energetischer Hinsicht, und es war verfahrenstechnisch einfach möglich, verdünnte Salpetersäuren einzusetzen. Dieses Verfahren wird mittlerweile auch großtechnisch ausgeübt (US 3 928 475, US 4 021 498, US 4 091 042, US 4 453 027, EP-A 436 443).

Das adiabatische Verfahren konnte auch auf die Herstellung von Dinitroaromaten angewendet werden (EP-A 597 361). Hier wird Toluol einstufig mit Nitriersäure zu DNT umgesetzt. Durch Verwendung von Nitriersäuren spezieller Zusammensetzung ist es möglich, den Nitrierprozeß adiabatisch zu betreiben und die Reaktionswärme im System zu konservieren. Ein Kühlen des Prozesses wie beim herkömmlichen isothermen Verfahren ist nicht mehr erforderlich, wodurch teure Kühlleistung eingespart wird. Nach Trennung der Phasen wird die heiße Absäure im Vakuum verdüst und die Reaktionswärme des Prozesses zur Aufkonzentrierung der Absäure genutzt. Aufgrund der adiabatischen Reaktionsführung und der damit verbundenen hohen Reaktionstemperatur ist der Prozeß zudem prädestiniert für den Einsatz von verdünnten Salpetersäuren. Je nach eingesetzter Salpetersäurequalität ist kein bzw. nur noch ein geringes Aufheizen im Aufkonzentrierschritt erforderlich.

Nachteilig an diesem Verfahren ist der hohe Anteil an ortho-DNT im Vergleich zur isothermen Fahrweise. Dies ist auf die höhere Reaktionstemperatur während der Nitrierung zurückzuführen. Ortho-DNT ist ein unerwünschtes Gemisch aus DNT-Isomeren mit ortho-ständigen Nitrogruppen (2,3-, 3,4-DNT), welches bei der TDI-Herstellung nicht verwendet werden kann. Es stellt daher ein Abfallprodukt dar und muß mit beträchtlichem Aufwand abgetrennt werden.

Auch das klassische, isotherme Nitrierverfahren liefert einen gewissen Anteil an ortho-DNT, allerding liegt dieser nicht so hoch wie beim adiabatischen Verfahren. Die Abtrennung der ortho-DNT-Isomeren erfolgt normalerweise nach der Hydrierung auf der Amin-Stufe und erfordert eine sehr gut trennende Kolonne, da sich der Siedepunkt des abzutrennenden Produktes nur wenig von dem des gewünschten Amins unterscheidet. Aus diesem Grunde ist das Einstellen eines hohen Rücklaufverhältnisses notwendig, was die Destillationskosten deutlich in die Höhe treibt. Da für das abtgetrennte ortho-Amin keine Verwendung existiert, ist ein Mehranfall an diesem Produkt einem Ausbeuteverlust gleichzusetzen. Darüberhinaus verursacht ein Mehranfall an ortho-DNT auch Zusatzkosten in der Hydrierung sowie bei der Vernichtung des unerwünschten Produktes.

Ein weiterer Nachteil des adiabatischen Nitrierverfahrens offenbart sich bei der Aufkonzentrierung der Absäure. Diese Absäure enthält gelöste Organika, im wesentlichen gelöstes DNT. Dieses ist wasserdampfflüchtig und wird bei der Aufkonzentrierung der Absäure weitgehend mit ausgedampft. Die modernen und sicheren Vakuumverfahren erfordern niedrige Kondensationsbedingungen für das verdampfte Wasser. Bei diesen Temperaturen kristallisiert DNT aus und führt zu Belägen im Kondensationssystem.

Dieses Problem besteht zwar auch beim isothermen Verfahren, hier behilft man sich jedoch durch Einspritzen von MNT in die heißen Brüdengase (DE-A 3 409 719). Auf diese Weise wird das Brüdenkondensat durch Schmelzpunkterniedrigung flüssig gehalten und Verstopfungen werden vermieden. Dieses ist beim bekannten adiabatischen Verfahren nicht möglich.

Aufgabe war es, die Dinitrierung von Aroamten, insbesondere von Toluol, dahingehend zu verbessern, daß ein niedriger Gehalt an ortho-DNT entsteht und darüberhinaus die obigen Probleme bei der Aufkonzentrierung der Absäure vermieden werden.

Diese Aufgabe konnte überraschenderweise durch das erfindungsgemäße zweistufige Verfahren gelöst werden.

Gegenstand der Erfindung ist ein zweistufiges Verfahren zur kontinuierlichen Herstellung von Dinitrotoluol-Isomerengemischen mit geringem ortho-DNT-Anteil durch Nitrierung von Toluol, welches dadurch gekennzeichnet ist, daß man in der ersten Stufe Toluol und Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Nitrotoluol-Isomeren und zum Rest aus Nebenprodukten bestehen, in einem kontinuierlich betriebenen Reaktor isotherm bei Temperaturen von 0 bis 100°C unter Einstellung eines Molverhältnisses von Salpetersäure: Toluol von mindestens 0,7:1 und höchstens 1,2:1 zur Reaktion bringt, anschließend die Phasen trennt, die in der ersten Stufe abgetrennte organische Phase (hauptsächlich Mononitrotoluol; MNT) unter adiabatischen Bedingungen bei Temperaturen von 20 bis 200°C, vorzugsweise 40 bis 180°C, besonders bevorzugt von 60 bis 170°C, mit einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Nitrotoluol-Isomeren und zum Rest aus Nebenprodukten bestehen, zur Reaktion bringt, wobei das Molverhältnis von Salpetersäure zu Mononitrotoluol mindestens 0,7:1 und höchstens 1,2:1 beträgt, anschließend die Phasen trennt und die Säurephase aus der zweiten Stufe durch Entspannungsverdampfung gegebenenfalls unter gleichzeitiger Wärmezufuhr von mindestens 5 % des Wassers destillativ befreit und nach Zugabe von 50 bis 100 Gew.-%iger Salpetersäure kontinuierlich in die Reaktion zurückführt.

Bevorzugt wird den Brüden aus der Aufkonzentrierung der Säurephase aus der zweiten Stufe Mononitrotoluol (MNT) aus der ersten Stufe vor deren Kondensation zugesetzt.

Die Menge an zugesetztem MNT wird so gewählt, daß das Brüdenkondenst flüssig abläuft und keine festen Beläge bildet. Dies ist der Fall, wenn in der organischen Phase des Brüdenkondensates ein Gewichtsverhältnis von MNT zu DNT von 2:1 bis 10:1 vorliegt. Die organischen Bestandteile des Brüdenkondensates werden nach Phasentrennung in die erste oder in die zweite Nitrierstufe zurückgeführt.

Die Absäure aus der ersten Stufe wird bevorzugt ebenfalls von mindestens 5 % des Wassers destillativ befreit und nach Zugabe von 50 bis 100 Gew.-%iger Salpetersäure kontinuierlich in die Reaktion zurückgeführt.

Überraschenderweise wurde gefunden, daß ein niedriger ortho-DNT-Gehalt erzielt wird, wenn die erste Stufe (zum Mononitrotoluol) isotherm und die zweite Stufe (von MNT zum Dinitrotoluol) adiabatisch geführt wird. Durch dieses erfindungsgemäße Verfahren werden ortho-DNT-Gehalte erzielt, die nicht über den Gehalten beim herkömmlich isotherm geführten Verfahren liegen (≦ 4,5 Gew.-%). Durch die adiabatische Fahrweise in der zweiten Stufe bleiben die Nutzung der Reaktionswärme im Prozeß und der Einsatz von verdünnter Salpetersäure erhalten.

Besonders vorteilhaft ist dieses Verfahren auch beim Umrüsten alter bestehender, zweistufiger, isothermer Nitrieranlagen auf die neuere adiabatische Technologie. Die bereits vorhandene isotherme erste Stufe kann voll genutzt werden, so daß nur die adiabatische zweite Stufe umzurüsten ist.

### Beispiel 1

92,14 g/h Toluol (1 mol/h) und 895 g/h (1,08 mol/h) Nitriersäure der Zusammensetzung 71,9:7,6:20,5 (Gew.-% H₂SO₄:HNO₃:H₂O) werden kontinuierlich in einem Reaktor bei 40°C unter Kühlung isotherm zur Reaktion gebracht. Die Phasen werden getrennt, die Absäure wird im Vakuum aufkonzentriert und nach Aufstockung mit 60%iger Salpetersäure in die Reaktion zurückgeführt. Die abgetrennte organische Phase wird unter adiabatischen Bedingungen mit 1890 g/h (1,08 mol/h) Nitriersäure der Zusammensetzung 77,9:3,6:18,5 (Gew.-% H₂SO₄:HNO₃:H₂O) bei einer Starttemperatur von ca. 120°C (resultiert durch Mischung mit der rückgeführten aufkonzentrierten Absäure des adiabatischen Nitrierschritts) kontinuierlich umgesetzt. Nach Trennung der Phasen wird die Säurephase im Vakuum aufkonzentriert. Um Ablagerungen im Kondensationsteil zu vermeiden, werden 9 g/h MNT aus der ersten Nitrierstufe in die überhitzten Brüden des Eindampfers gegeben. Die aufkonzentrierte Absäure wird nach Aufstockung mit 60%iger Salpetersäure in den adiabatischen Nitrierschritt zurückgeführt, ebenso die organischen Bestandteile des Brüdenkondensates. Es werden 180 g/h (99 Gew.-%) Dinitrotoluolisomerengemisch isoliert. Der Gehalt an ortho-DNT beträgt 4,1 Gew.-%.

### Beispiel 2

92,14 g/h Toluol (1 mol/h) und 1063 g/h (1,08 mol/h) Nitriersäure der Zusammensetzung 72,5:6,4:21,1 (Gew.-% H₂SO₄:HNO₃:H₂O) werden kontinuierlich in einem Reaktor bei 40°C unter Kühlung isotherm zur Reaktion gebracht. Die Phasen werden getrennt, die Absäure wird im Vakuum aufkonzentriert und nach Aufstockung mit 60%iger Salpetersäure in die Reaktion zurückgeführt. Die abgetrennte organische Phase wird unter adiabatischen Bedingungen mit 586,6 g/h (1,08 mol/h) Nitriersäure der Zusammensetzung 73,6:11,6:14,8 (Gew.-% H₂SO₄:HNO₃:H₂O) bei einer Starttemperatur von ca. 60°C kontinuierlich umgesetzt. Nach Trennung der Phasen wird die Säurephase im Vakuum aufkonzentriert. Um Ablagerungen im Kondensationsteil zu vermeiden, werden 4,5 g/h MNT aus der ersten Nitrierstufe in die überhitzten Brüden des Eindampfers gegeben. Die aufkonzentrierte Absäure wird nach Aufstockung mit 98,5%iger Salpetersäure in den adiabatischen Nitrierschritt zurückgeführt, ebenso die organischen Bestandteile des Brüdenkondensates. Es werden 180 g/h (99 Gew.-%) Dinitrotoluolisomerengemisch isoliert. Der Gehalt an ortho-DNT beträgt 4,0 Gew.-%.

### Beispiel 3

92,14 g/h Toluol (1 mol/h) und 895 g/h (1,08 mol/h) Nitriersäure der Zusammensetzung 71,9:7,6:20,5 (Gew.-% H₂SO₄:HNO₃:H₂O) werden kontinuierlich in einem Reaktor bei 40°C unter Kühlung isotherm zur Reaktion gebracht. Die Phasen werden getrennt, die Absäure wird im Vakuum aufkonzentriert und nach Aufstockung mit 60%iger Salpetersäure in die Reaktion zurückgeführt. Die abgetrennte organische Phase wird unter adiabatischen Bedingungen mit 1173,1 g/h (1,08 mol/h) Nitriersäure der Zusammensetzung 76,9:5,8:17,3 (Gew.-% H₂SO₄:HNO₃:H₂O) bei einer Starttemperatur von ca. 100°C kontinuierlich umgesetzt. Nach Trennung der Phasen wird die Säurephase im Vakuum aufkonzentriert. Um Ablagerungen im Kondensationsteil zu vermeiden, werden 7 g/h MNT aus der ersten Nitrierstufe in die überhitzten Brüden des Eindampfers gegeben. Die aufkonzentrierte Absäure wird nach Aufstockung mit 68%iger Salpetersäure in den adiabatischen Nitrierschritt zurückgeführt, ebenso die organischen Bestandteile des Brüdenkondensates. Es werden 180 g/h (99 Gew.-%) Dinitrotoluolisomerengemisch isoliert. Der Gehalt an ortho-DNT beträgt 4,1 Gew.-%.

## Patentansprüche

1. Zweistufiges Verfahren zur kontinuierlichen Herstellung von Dinitrotoluol-Isomerengemischen durch Nitrierung von Toluol, dadurch gekennzeichnet, daß man in der ersten Stufe Toluol und Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Nitrotoluol-Isomeren und zum Rest aus Nebenprodukten bestehen, in einem kontinuierlich betriebenen Reaktor isotherm bei Temperaturen von 0 bis 100°C unter Einstellung eines Molverhältnisses von Salpetersäure:Toluol von mindestens 0,7:1 und höchstens 1,2:1 zur Reaktion bringt, anschließend die Phasen trennt, die in der ersten Stufe abgetrennte organische Phase unter adiabatischen Bedingungen bei Temperaturen von 20 bis 200°C mit einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Nitrotoluol-Isomeren und zum Rest aus Nebenprodukten bestehen, zur Reaktion bringt, wobei das Molverhältnis von Salpetersäure zu Mononitrotoluol mindestens 0,7:1 und höchstens 1,2:1 beträgt, anschließend die Phasen trennt, die Säurephase durch Entspannungsverdampfung gegebenenfalls unter gleichzeitiger Wärmezufuhr von mindestens 5 % des Wassers destillativ befreit und nach Zugabe von 50 bis 100 Gew.-%iger Salpetersäure kontinuierlich in die Reaktion zurückführt.

## Claims

1. Two-stage process for the continuous production of dinitrotoluene isomer mixtures by toluene nitration, characterised in that in the first stage toluene and nitrating acid comprising (i) from 80 to 100 wt.% inorganic constituents which are composed substantially of from 60 to 90 wt.% sulfuric acid, from 1 to 20 wt.% nitric acid and at least 5 wt.% water and (ii) from 0 to 20 wt.% organic constituents which comprise 70% to 100% nitrotoluene isomers and for the remainder by-products, are reacted isothermally in a continuously operated reactor at temperatures of from 0 to 100°C, with a molar ratio of nitric acid : toluene of at least 0.7 : 1 and a maximum of 1.2 : 1 being set, followed by phase separation, the organic phase which is separated in the first stage is reacted under adiabatic conditions at temperatures of from 20 to 200°C with a nitrating acid comprising (i) from 80 to 100 wt.% inorganic constituents which are composed substantially of from 60 to 90 wt.% sulfuric acid, from 1 to 20 wt.% nitric acid and at least 5 wt.% water and (ii) from 0 to 20 wt.% organic constituents which comprise 70% to 100% nitrotoluene isomers and for the remainder byproducts, wherein the molar ratio of nitric acid : mononitrotoluene is at least 0.7 : 1 and a maximum of 1.2 : 1, followed by phase separation, and at least 5% of the water is removed by distillation by flash evaporation from the acid phase from the second stage, optionally with simultaneous supply of heat, which acid phase is recycled continuously into the reaction after the addition of 50 to 100 wt.% nitric acid.

## Revendications

1. Procédé en deux étapes pour la préparation continue de mélanges d'isomères de dinitrotoluène par nitration de toluène, caractérisé en ce que l'on fait réagir dans la première étape du toluène et un mélange sulfonitrique constitué (i) de 80 à 100 % en poids de constituants inorganiques, qui sont constitués essentiellement de 60 à 90 % en poids d'acide sulfurique, de 1 à 20 % en poids d'acide nitrique et d'au moins 5 % en poids d'eau et (ii) de 0 à 20 % en poids de constituants organiques qui sont constitués jusqu'à de 70 à 100 % d'isomères de nitrotoluène et le reste étant constitué de produits secondaires, dans un réaction fonctionnant en continu de manière isotherme à des températures de 0 à 100°C en ajustant un rapport molaire acide nitrique : toluène d'au moins 0,7 : 1 et d'au plus 1,2 : 1, on sépare ensuite les phases, on fait réagir la phase organique séparée dans la première étape dans des conditions adiabatiques à des températures de 20 à 200°C avec un mélange sulfonitrique constitué (i) de 80 à 100 % en poids de constituants inorganiques qui sont essentiellement constitués de 60 à 90 % en poids d'acide sulfurique, de 1 à 20 % en poids d'acide nitrique et d'au moins 5 % en poids d'eau et (ii) de 0 à 20 % en poids de constituants organiques, lesquels sont constitués jusqu'à de 70 à 100 % en poids d'isomères de nitrotoluène et le reste étant constitué de produits secondaires, le rapport molaire de l'acide nitrique au mononitrotoluène étant d'au moins 0,7 : 1 et d'au plus 1,2 : 1, on sépare ensuite les phases, on libère par distillation la phase acide d'au moins 5 % de l'eau par distillation éclair éventuellement avec un apport simultané de chaleur et on la renvoie en continu dans la réaction après addition de 50 à 100 % en poids d'acide nitrique.
